# EUROPEAN PATENT SPECIFICATION

(11) **EP 2 226 071 B1**
(45) Date of publication and mention of the grant of the patent: **13.04.2016**
(21) Application number: 08867917.0
(22) Date of filing: 28.08.2008
(51) Int. Cl.: A61K 31/045, A23K 20/20, A23L 33/00, A23L 33/15, A61K 31/164, A61K 36/00, A61P 3/06, A61P 3/04

(54) **COMPOSITION FOR ORAL ADMINISTRATION**
ZUSAMMENSETZUNGEN ZUR ORALEN VERABREICHUNG
COMPOSITION POUR ADMINISTRATION ORALE

(30) Priority: 28.12.2007 JP 2007339460; 01.02.2008 JP 2008022784
(43) Date of publication of application: 08.09.2010
(73) Proprietor: Daicel Corporation, Osaka-shi, Osaka 530-0011 (JP)
(72) Inventor: MUKAI, Katsuyuki, Uji-shi, Kyoto 611-0021 (JP); SHIRAKURA, Yoshiyuki, Uji-shi, Kyoto 611-0021 (JP)
(74) Representative: Hoffmann Eitle
(86) International application number: PCT/JP2008/065466
(87) International publication number: WO 2009/084275

(56) References cited:
- EP-A1- 1 772 143
- WO-A1-2005/112904
- WO-A1-2006/068041
- WO-A1-2007/116981
- WO-A2-2004/112776
- JP-A- 1 279 837
- JP-A- 2006 083 151
- JP-A- 2006 089 452
- JP-A- 2006 104 088
- JP-A- 2006 219 388
- JP-A- 2006 325 602
- JP-A- 2007 091 693
- JP-A- 2007 223 914
- TAKAO SASAKI: '''Unshu Mikan Extract 'Cript Beta '''' FOOD STYLE 21 vol. 11, no. 12, 01 December 2007, pages 63 - 65
- KEIZO SEKIYA: 'Kankitsu, Momo no Seibun ni yoru Insulin Kanjusei no Josho' KAJU KYOKAIHO vol. 497, 2000, pages 30 - 34
- KEIZO SEKIYA: 'Shibo Saibo wa Akudama ka? - Shibo Saibo o Chiisaku suru Nosakumotsu Seibun' AGRICULTURE AND HORTICULTURE vol. 81, no. 3, 2006, pages 335 - 343

## Description

### Technical Field

The present invention relates to a composition having high safety, containing a composition containing a carotenoid and a sphingolipid and/or a derivative thereof, which has a body fat reducing action, and can be ingested as a food, a pharmaceutical or a feed.

### Background of the Invention

In Japan, undertaking practice of medical examination and insurance guidance, which aims at metabolic syndrome has been obligated for an insured person and a supported person of 40 years old or older, among the people who receive a medical insurance (national health insurance, employee insurance), since April, 2008. By judging the results 5 years thereafter, a financial penalty is imposed on an insured person having poor results. Ministry of Health, Labor and Welfare expects that one of two middle-aged men is undergoing metabolic syndrome, and about twenty million people are under metabolic syndrome and reserve group thereof.

In recent years, with the advance of studies on the influence of fat on the body, it has been suggested that accumulation of visceral fat in particular has high correlation with not only obesity but life-style-related disease, hyperlipemia, sugar resistance abnormality, high blood pressure and the like, and it is considered that not allowing visceral fat to be accumulate leads to the prevention of many diseases. A pathology based on these considerations is metabolic syndrome. In order to reduce visceral fat, life environment must be changed such as improving diet and taking daily exercise. However, it is the situation that diet becomes high calories due to westernized diet and change in life style, and it is difficult to ensure a time for taking exercise.

With the current advance of age in Japan, a preventive medical treatment is drawing attention in order to suppress the increasing medical expenses. Since the above-mentioned diseases require a large amount medical fees and treating time when generated, various health foods and supplements are on sale with the aim of alleviating onset risk of the diseases by reducing visceral fat from a preventive medical treatment point of view.

However, it is difficult to expect sufficient effects from these health foods by general ingestion, and although a certain degree of effects can be expected when ingested in a large amount, it poses a disadvantage of increasing side effects. In view of the above, great concern has been directed toward the development of a food which is effective with a small amount of ingestion and shows a safe visceral fat reducing action.

As the existing food having such an effect to reduce body fat, particularly visceral fat, catechin and astaxanthin can be mentioned. Catechin is contained in green tea, tea, chocolate and the like, and considered to be effective in combusting body fat (Patent Reference 1). However, since astringency is increased when the content is increased, its addition to commodities is largely limited. In addition, astaxanthin also shows the effect to combust body fat (Patent Reference 2), but since astaxanthin itself can hardly be up-taken into the body and its effect by individual use is not high, it is difficult to ingest its effective amount for reducing fat.

In addition, there are reports on tests using culture cells in which obesity can be prevented by β-cryptoxanthin alone (Patent Reference 3 and Non-patent Reference 1), but when we have tested, its action by individual use was weak so that it was difficult to ingest its effective amount for reducing fat.

WO 2004/112776 relates to a composition comprising a source of long chain polyunsaturated fatty acids, for example, docosahexaenoic acid (DHA), and a carotenoid, for example, astaxanthin, and other nutrients for prophylactic and/or therapeutic use in the healing of trauma- and stress-induced inflammatory condition.

WO 2005/112904 is intended to provide a peroxisome proliferator-activated receptor (PRAR) activator, such as beta-cryptoxanthin from citrus fruits, which is free from the problem of side effects, can be taken over a long term and has no characteristic taste.

The use of ceramides or of milk phospholipids for preventing excessive body fat is disclosed in WO 2006/06841 and WO 2007/116981, respectively. JP 2006 219388 A relates to a citrus pulp containing functional materials, and more particularly, citrus pulp and/or citrus-derived lipid metabolism improving agent containing the substance and its food, or cholesterol metabolism improving agent and its food, glucose tolerance and/or it relates to the insulin sensitizer and the food.
Patent Reference 1: JP-A-2002-326932
Patent Reference 2: JP-A-2007-153845
Patent Reference 3: International Publication No. 2005/112904

Non-patent Reference 1: Abstracts of papers, 28th Japanese Society of Obesity, p. 288

### Disclosure of the Invention

### Problems that the Invention is to Solve

By taking the above-mentioned present situation into consideration, great concern has been directed toward the provision of a food, a pharmaceutical and a feed having an action to reduce body fat such as visceral fat and subcutaneous fat with a general ingesting amount. The present invention aims at providing a food, a pharmaceutical and a feed showing high safety and having a body fat reducing action.

### Means for Solving the Problems

In order to solve such problems, the present inventors have carried out intensive studies and found as a result that, when a carotenoid and a sphingolipid and/or a derivative thereof are used in combination, effects of more than the functionality which can be expected when used alone can be exerted by their synergistic action, thus accomplished the present invention. In addition, it was found that when citrus fruits plants are used as the materials in the combination use, their extraction and concentration can be carried out easily.

That is, the first embodiment of the present invention relates to an oral administration composition having a body fat reducing action which comprises a carotenoid and a sphingolipid, wherein the carotenoid is cryptoxanthin and/or a fatty acid ester thereof, and those wherein the carotenoid and/or the sphingolipid are derived from a citrus fruit and preferably the citrus fruit is *Citrus unshiu.*

The method for producing any of the above-mentioned compositions, (1) comprises squeezing a citrus fruit plant and obtaining a composition comprising a carotenoid and/or a sphingolipid from the resulting residue, (2) comprises obtaining a composition comprising a carotenoid and/or a sphingolipid by treating a citrus fruit plant with an enzyme, or (3) comprises allowing a citrus fruit plant to contact with an organic solvent and extracting a composition comprising a carotenoid and/or a sphingolipid into the organic solvent.

The gist of one embodiment of the present invention resides in a food, a pharmaceutical or a feed which comprises the above-mentioned oral administration composition.

The gist of one embodiment of the present invention resides in the above oral administration composition for use in a method for reducing a body fat, which comprises administering the above-mentioned oral administration composition to an object.

The gist of one embodiment of the present invention resides in use of the above-mentioned oral administration composition for the manufacture of an agent for reducing a body fat.

### Effect of the Invention

According to the present invention, which is defined by the claims, because of the high body fat reducing action and high safety, it becomes possible to blend in various foods, pharmaceuticals and feeds. In addition, since the action can be verified with a small amount, an action effect of being easy in the blend designing can be obtained.

### Brief Description of the Drawings

Fig. 1 is a graph showing a change in the abdominal fat area at the L4/L5 crossing region.
Fig. 2 is a graph showing a change in the subcutaneous fat area at the L4/L5 crossing region.
Fig. 3 is a graph showing a change in the total fat area at the L4/L5 crossing region.
Fig. 4 is a graph showing a change in the body weight.
Fig. 5 is a graph showing a change in the waist.
Fig. 6 is a graph showing a change in the neutral fat (TG).
Fig. 7 is a graph showing a change in the body weight.
Fig. 8 is a graph showing a change in the amount of serum triglyceride.
Fig. 9 is a graph showing a change in the weight of visceral fat.
Fig. 10 is a graph showing a change in the body weight.
Fig. 11 is a graph showing a change in the blood triglyceride.
Fig. 12 is a graph showing a change in the weight of visceral fat.
Fig. 13 is a graph showing a change in the body weight.
Fig. 14 is a graph showing a change in the blood triglyceride.
Fig. 15 is a graph showing a change in the weight of visceral fat.
Fig. 16 is a graph showing a change in the body weight.
Fig. 17 is a graph showing a change in the blood triglyceride.
Fig. 18 is a graph showing a change in the weight of visceral fat.
Fig. 19 is a graph showing a change in the body weight.
Fig. 20 is a graph showing a change in the blood triglyceride.
Fig. 21 is a graph showing a change in the weight of visceral fat.
Fig. 22 is a graph showing a change in the body weight.
Fig. 23 is a graph showing a change in the blood triglyceride.
Fig. 24 is a graph showing a change in the weight of visceral fat.

In this connection, in the drawings, * represents p < 0.05, and ** represents p< 0.01.

### Best Mode for Carrying Out the Invention

The following describes the present invention in detail, which invention is defined by the claims.

In the present specification, unless otherwise noted, all of the ratio, percentage and the like are based on mass, and this is the same as those based on weight.

The carotenoid in the present invention is, cryptoxanthin, and/or fatty acid esters thereof. As the fatty acid esters, there may be mentioned palmitoyl esters, myristoyl esters, lauryl esters and the like. Cryptoxanthin and a fatty acid ester thereof are desirable because of their high body fat reducing action.

The cryptoxanthin in the present invention is not particularly limited, and for example, α-cryptoxanthin, β-cryptoxanthin, a fatty acid ester compound thereof and a structurally acceptable derivative thereof are also included. Fatty acid length of the fatty acid ester compound is not particularly limited, either, and for example, there may be mentioned fatty acid esters of lauric acid (C12), myristic acid (C 14), palmitic acid (C16), stearic acid (C18) and the like. In addition, as the supply source of these cryptoxanthins, citrus fruits are desirable, and a *Citrus unshiu* (*Citrus unshiu* Mark.) origin, which is an old time fruit in Japan and has high production, is particularly desirable.

The sphingolipid in the present invention is a group of compounds, in which an amino-alcohol which is so-called sphingosine consisting of approximately from 14 to 24 carbon atoms or a long chain amino-alcohol having a structure similar to this, is amido-bonded with a fatty acid such as a long chain fatty acid or the like, and compounds in which a saccharide is bonded thereto, compounds in which a phosphate group is bonded thereto, and the like, are also included. The fatty acid may be either a saturated fatty acid or an unsaturated fatty acid, may be a straight chain or a branched chain and may be substituted with one or more hydroxyl groups. In addition, a sugar, phosphoric acid, sialic acid, choline, ethanolamine or the like may be linked to the hydroxyl group of amino-alcohol. As the saccharides, it is desirable that glucose, mannose, galactose, lactose and the like are bonded thereto, and particularly, it is further desirable that glucose is bonded thereto. In the case in which phosphate group is bonded, a sphingolipid to which inositol, further a sugar, are bonded via the phosphate group is also included. The present invention does not use those having a specific chain length or structure as the object, and all of the substances which are called sphingolipid are included. In addition, its origin may be a natural product origin or a conventionally known sphingolipid and a derivative thereof obtained by chemical synthesis. When these sphingolipids are used for a food, those which are derived from rice, wheat, milk, corn, beet, *konnyaku,* apple, various mushrooms such as *Pleurotus cornucopiae* and *Grifola frondosa,* acetic acid bacteria and the like can be obtained, so that it is possible to use them as such, but a sphingolipid derived from citrus fruits is preferable, and the sphingolipid derived from *Citrus unshiu* is particularly preferable. Since the sphingolipid derived from citrus fruits contains free sphingolipid which does not include sugar in a large amount and therefore has strong body fat reducing, it is desirable to use these origins.

As the citrus fruits in the present invention, plants belonging to the family Rutaceae and the like can be mentioned. In the present invention, the cryptoxanthin carotenoid and/or the sphingolipid is derived from a citrus fruit selected from *iyokan* (*Citrus iyo*), *Citrus unshiu,* orange, *kabosu (Citrus sphaerocarpa*), *kawabata, kishu mikan (Citrus kinokuni*), *kiyomi, kinkan* (*Citrus fortunella*), grape fruit, *gekkitsu* (*Murraya paniculata*), *sanpokan* (*Citrus sulcata*), *shekwasha* (*Citrus depressa Hayata*), *jabara* (*Citrus jabara*), sweety (oroblanco), *sudachi* (*Citrus sudachi*), *daidai* (*Citrus · sinensis* var. daidai), *tachibana* (*Citrus tachibana*), *dekopon, natsu daidai, hassaku* (*Citrus hassaku*), Valencia orange, *banpaku yuzu* (*Citrus grandis, Citrus maxima*), *hyuga natsu* (*Citrus tamurana* hort. ex T. Tanaka), *buntan* (*Citrus maxima*), *ponkan* (*Citrus reticulata*), mandarin orange, *yatsushiro, yuzu* (*Citrus junos*), lime (*Citrus aurantifolia*)*, , karatachi* (*Poncirus trifoliate*) and the like can be exemplified.

The oral administration composition of the present invention may contain a substance other than a carotenoid and a sphingoid base, which has been reported to have an effect to reduce visceral fat. As the blending amounts in that case, it is desirable to blend from 0.01 to 90% (mass ratio) as the amount of the substance other than a carotenoid and a sphingoid lipid. As the substance, having the effect to reduce visceral fat, conventionally known substances may be used. For example, it is desirable to blend an ω3 unsaturated fatty acid, fucoxanthin, L-arabinose, DHA, EPA, capsaicin, L-carnitine and the like, alone or by a combination thereof.

When orally ingested, the oral administration composition of the present invention described above shows the action to reduce body fat such as visceral fat, subcutaneous fat and the like, neutral fat typified by triglyceride, and the like.

Regarding the method for ingesting the oral administration composition of the present invention, the composition may be directly ingested as such alone or may be ingested by processing into powders, tablets, granules, capsules, soft capsules, gels, pastes, syrups, suspensions, emulsions, drinks and the like.

Next, production method of the above-mentioned oral administration composition having a body fat reducing action is described. As such a production method, there may be mentioned a method in which a citrus fruit plant is squeezed, and a composition containing a carotenoid, and a sphingolipid and/or a derivative thereof are obtained from the residue; a method in which a composition containing a carotenoid, and a sphingolipid and/or a derivative thereof are obtained by enzyme-treating a citrus fruit plant through the addition of an enzyme; and a method in which an organic solvent is added to a citrus fruit plant, and a composition containing a carotenoid, and a sphingolipid and/or a derivative thereof is extracted into the organic solvent. These methods are explained below in this order.

Regarding the citrus fruit plant to be used as a material for extraction and enzyme treatment, parts of the citrus fruit plant do not matter, but among them, it is desirable to use its fruits as a part having the high content. Fruits of the citrus fruit plant may be used as such or after carrying out their physical treatments such as mashing, crushing, pulverization, heating, dehydration, drying and the like. It is desirable to carry out these treatments because the enzyme treatment efficiency and extraction efficiency are increased. In addition, according to the present invention, it is desirable to use the squeezed residue of fruits. The squeezed residue of fruits is prepared by squeezing the fruits using an inline squeezer, a chopper helper squeezer, a Brown squeezer and the like, preparing fruit juice by its filtration or screening using a paddle type or screw type finisher or the like or its centrifugation, and then collecting the resulting squeezing residue. In addition, also as the squeezed residue of citrus fruit plant to be used in the present invention is a squeezed residue, which is minute pulp content, obtained by a centrifugation treatment of the fruit juice after squeezing by a squeezer. Such minute pulp content is a suitable material because it further has the high content of a composition containing a carotenoid and a sphingolipid and/or a derivative thereof.

Regarding the organic solvent to be used in the extraction process in the present invention, it may be any substance, with the proviso that a fraction having a body fat reducing action can be obtained from a citrus fruit or processed product thereof as the material and does not spoil the present invention. In addition, one kind of solvent may be used alone or two or more solvents may be used by mixing them. As such solvents, there may be mentioned for example, alcohols such as methanol, ethanol, propanol, isopropanol and the like, polyhydric alcohols such as ethylene glycol, propylene glycol, butylene glycol, glycerol and the like, ketones such as acetone, methyl ethyl ketone and the like, esters such as methyl acetate, ethyl acetate and the like, halogenated hydrocarbons such as acetonitrile, dichloromethane, dichloroethane, chloroform and the like, aliphatic hydrocarbons such as pentane, hexane and the like, aromatic hydrocarbons such as benzene, toluene and the like, ethers such as tetrahydrofuran, diethyl ether and the like, pyridines, polyethers such as polyethylene glycol and the like, supercritical carbon dioxide and the like. Among these, it is particularly desirable to use acetone, ethanol, hexane and supercritical carbon dioxide from the viewpoint of safety because these are used for a food application in the present invention. These organic solvents may be used alone or as a mixture thereof. In addition, additives such as water, a surfactant and the like can also be added to the organic solvents.

The amount of the organic solvent to be used in the extraction is not particularly limited with the proviso that it is an amount sufficient enough for extracting a cryptoxanthin and a sphingolipid from the substance to be extracted. Illustratively, it may be from 0.5 to 100 times, preferably from 1 to 50 times, based on the solid content weight of the substance to be extracted. Though it depends on the boiling point of the solvent to be used, in the case of the use of ethanol for example, the extraction temperature is preferably from 0°C to 80°C. When the extraction temperature is lower than this range, the extraction efficiency is lowered, and when higher than this range, it causes evaporation of the solvent, and only increasing the amount of the energy to be used. The extraction time is not particularly limited, but it is preferably from 10 minutes to 24 hours from the viewpoint of extraction efficiency and workability.

In this connection, the extraction operation is not limited to only one time of batch operation. The extraction operation can be carried out by adding a new solvent to the residue again after extraction, or the extraction solvent can be contacted two or more times with the substance to be extracted. That is, as the extraction operation, it is possible to use any one of the batch operation, semi-continuous operation and counter-current multiple step contact operation. In addition, conventionally known extraction methods such as the Soxhlet extraction, reflux extraction and the like may be used. Separation and removal of the residue after extraction may also be carried out by a conventionally known method, and illustratively, a suction filtration, a filter press, a cylinder press, a decanter, a centrifuge, a filtration centrifuge and the like may be used. Further, impurities may be subsequently removed. As the impurity removing method, there may be mentioned for example water washing, organic solvent washing, a method for passing through a silica gel column, resin column, reverse-phase column or the like, activated carbon treatment, partition by solvents having different polarities, recrystallization method, vacuum distillation method and the like.

The extract obtained in this manner can be used in all kinds of foods, pharmaceuticals and feeds by subsequently carrying out treatments such as alkali treatment, concentration, purification by a column or the like, drying, powderization and mixing with a carrier, emulsifier and the like.

In the present invention, the enzyme to be used for producing the enzyme-treated product of a citrus fruit plant is not particularly limited, with the proviso that it can degrade organic matters contained in citrus fruits, particularly biopolymers and the like which constitute cell wall and the like, and amylase, glucoamylase, cellulase, hemicellulase, pectinase, mannanase, xylanase, protease, peptidase, lipase, maceration enzyme (cell wall disintegrating enzyme) and the like are used. Among them, cellulase, hemicellulase, pectinase, mannanase, xylanase and maceration enzyme as glycosides are suitable enzymes because of their high efficiency to concentrate sphingolipid. As the enzyme preparation to be added, purified enzymes thereof may be used or microbial cells or culture mixture showing these activities or their crudely purified products may be used. In addition, commercially available enzymes can also be used, and for example, Sumizyme PX (manufactured by Shinnihon Chemicals Corporation), Sumizyme SPC (manufactured by Shinnihon Chemicals Corporation), Pectinex SRL (manufactured by Novozymes Japan Ltd.), Sumizyme PMAC (manufactured by Shinnihon Chemicals Corporation) and the like may be used as the pectinase; Cellulosine GM 5 (manufactured by HBI Enzyme Inc.), Cellulosine HC (manufactured by HBI Enzyme Inc.) and the like may be used as the hemicellulase; Cellulase Y-NC (manufactured by Yakult Pharmaceutical Industry Co., Ltd.), Entilon MCH (manufactured by Rakuto-Kasei Industry Co., Ltd.), Cellulase R10 (manufactured by Yakult Pharmaceutical Industry Co., Ltd.), Sumizyme CAP (manufactured by Shinnihon Chemicals Corporation), Cellulase TP-3 (manufactured by Kyowa Kasei Co., Ltd.) and the like may be used as the cellulase; and Protease M (manufactured by Amano Enzyme Inc.), Orientase 20A (manufactured by HBI Enzyme Inc.) and the like may be used as the protease.

As the enzyme preparation to be added, purified enzymes thereof may be used or microbial cells or culture mixture showing these activities or their crudely purified products may be used. These enzymes may be used alone or may be used by mixing two or more enzymes. Amount of the enzyme to be added is not particularly limited, and may be added in response to the reactivity of the enzyme. For example, in the case of the use of pectinase, it is preferably from 1 to 100,000 units, further preferably from 10 to 10,000 units, based on 100 g of the squeezed residue.

After adding of the above-mentioned enzymes, the enzyme reaction is advanced by uniformly mixing the enzymes and the substance to be extracted by stirring and the like. Regarding the reaction temperature in this case, it is desirable to carry out under such a condition that the enzymes are not inactivated and putrefaction hardly occurs, and such a condition that a sphingolipid and a carotenoid and a derivative thereof are not lost. Illustratively, the temperature is from 0°C to 90°C, preferably from 0°C to 80°C, further preferably from 0°C to 70°C. Regarding the reaction pH, it goes without saying that it is desirable to carry out under optimum condition of the enzyme, and it may be set to from pH 2 to 12, preferably from pH 2.5 to 8. The reaction time depends on the amounts of the squeezed residue and enzymes to be used, but it is generally suitable from the working point of view to set it to from 1 to 48 hours. In carrying out the reaction, the reaction may be carried out while stirring this reaction mixture, or it may be a static reaction.

After completion of the enzyme treatment, the enzyme-treated reaction mixture may be used as such or those to which a certain processing is applied may be used. Illustratively, a residue prepared by solid-liquid separating the reaction mixture, a preparation of drying the solid-liquid separated residue, a preparation of drying the reaction mixture as such without carrying out its solid-liquid separation, and the like may be used. Also, since impurities can be easily removed, it is desirable to use a water washing method which can easily remove the reaction products such as sugars formed by the enzyme treatment, by solid-liquid separating the enzyme-treated reaction mixture, further adding water and then again carrying out solid-liquid separation. In addition, the impurities may be further removed. As the method for removing impurities, there may be mentioned for example chemical treatments such as water washing or organic solvent washing, alkali treatment or the like, a method for passing through a silica gel column, resin column, reverse-phase column or the like, activated carbon treatment, partition by solvents having different polarities, recrystallization method, vacuum distillation method and the like.

The composition of the present invention obtained in the above manner exerts various functionalities, and particularly among them, shows the action to reduce body fat such as visceral fat, subcutaneous fat and the like, neutral fat typified by triglyceride and the like, by oral administration.

As the method for ingesting the composition of the present invention, the composition may be ingested alone as such or may be ingested by processing into powders, tablets, granules, capsules, soft capsules, gels, pastes, syrups, suspensions, emulsions, drinks and the like. A standard of the amount to be ingested in the case of ingestion may be optionally selected based on the age, sex, body weight and the like of each object, but in general, in the case of an adult, the daily dose is (1) from 0.01 mg to 1 g of a carotenoid and from 0.01 mg to 10 g of sphingolipid, or (2) from 0.01 mg to 1 g of a carotenoid and/or a derivative thereof. Desirable dose is (1) from 0.1 mg to 100 mg of a carotenoid and from 0.1 mg to 1 g of sphingolipid, and/or a derivative thereof.

The period by which the effect of the present invention can be obtained by ingesting the composition of the present invention is not particularly limited, but it is desirable that the number of ingesting days per year is 1 day or more and 365 days or less, preferably from 14 days or more and 365 days or less, further preferably from 60 days or more and 365 days or less.

The object to whom the composition is administered in the present invention, is not particularly limited as long as its effect is exerted, but is preferably a warm-blooded animal, further preferably a mammal, most preferably human.

Another embodiment of the present invention is a food or beverage and a pharmaceutical, which comprise the above-mentioned composition of the present invention. Regarding the pharmaceutical, it can be ingested in the form of injections, transfusions, powders, tablets, granules, capsules, pills, enteric preparations, suspensions, syrups, oral liquids, troches, emulsions, liquids for external use, fomentations, nasal drops, ear drops, eye drops, inhalations, ointments, lotions, suppositories, intestinal nutrients and the like. These can be used alone or in combination of two kinds or more depending on the symptoms. Containing amount of the composition of the present invention may be optionally decided within a range of from 0.1% to 90%, but it is desirable to carry out preparation designing in such a manner that the above-mentioned standard ingesting amount per day can be ingested.

In addition, the food or beverage of the present invention includes, in addition to a general food, all foods and/or beverages such as food for specified health uses, health foods, functional foods, quasi drugs and the like. The food and/or beverage are not particularly limited, and for example, in addition to the above-mentioned pharmaceutical-like embodiments, there may be mentioned staple foods such as bread, noodles, buckwheat noodles, boiled rice and the like, foods such as cheese, Vienna sausage, sausage, ham, fishery processed products and the like, confectionary such as ice cream, cookie, cake, jelly, pudding, candy, chewing gum, yogurt, gummy, chocolate, cracker and the like, soft drink, condiments such as jam and the like, and beverages such as alcoholic liquors, nutritious drink, coffee, tea, milk, fruit drink, soft drink and the like. Containing amount of the composition of the present invention may be optionally decided within a range of from 0.1 % to 70%, but it is desirable to decide the blending amount in such a manner that the above-mentioned standard of ingesting amount per day can be ingested.

As the feed, it can be produced by blending the composition of the present invention with, for example, cereals such as corn, wheat, barley, rye and the like, brans such as wheat bran, rice bran and the like, strains such as corn gluten meal, corn germ meal and the like, animal feeds such as skimmed dry milk, whey, fish powder, bone powder and the like, yeasts such as beer yeast and the like, calciums such as calcium phosphate, calcium carbonate and the like, vitamins, oils and fats, amino acids, saccharides and the like. As a form of the feed, it can be used in a pet food, a livestock feed, a farming fish feed and the like. Containing amount of the composition may be optionally decided within a range of from 0.01 to 20%, but it is desirable to add to a feed material and the like in such a manner that (1) from 0.001 mg to 100 mg of a carotenoid and from 0.01 mg to 1 g of sphingolipid, and/or a derivative thereof, per day per 1 kg of body weight, can be ingested.

### Examples

The following describes examples of the tests. In this connection, the present invention does not limit its scope by the examples. A high performance liquid chromatography (HPLC) was used for the measurement of cryptoxanthin. Using LC-10A (manufactured by Shimadzu Corporation) as the HPLC device, a Resolve C18 (φ3.9 x 150 mm, manufactured by Waters Corporation) column was connected thereto, and a sample mixed with the same volume of methanol was introduced. Methanol:ethyl acetate = 7:3 was used as the mobile phase and analyzed at a column temperature of 30°C, a flow rate of 1.0 ml/min and detection wavelength of 450 nm.

The HPLC device LC-10A, to which an InertsilSIL 100A column (manufactured by GL Sciences Inc.) was connected, was used in the measurement of sphingolipid, and detected using a light scattering detector (500 ELSD, manufactured by ALLTECH). A linear gradient by two liquids of chloroform and chloroform/methanol (1/1) was used as the mobile phase and measured at 37°C at a flow rate of 1.0 ml/min.

Measurement of the containing amount of the flavonoid and/or a derivative thereof was carried out using a high performance liquid chromatography (HPLC). That is, using LC-10A (manufactured by Shimadzu Corporation) as the HPLC device, an Asahipak ODP-50 (φ4.6 x 150 mm, manufactured by Asahi Kasei Corporation) column was connected thereto, and a sample dissolved in 0.1 N NaOH was diluted 10 times with distilled water and introduced. Water:acetonitrile = 8.2 was used as the mobile phase and analyzed at a column temperature of 30°C, a flow rate of 1.5 ml/min and detection wavelength of 285 nm.

### Example 1

A fruit juice prepared by squeezing *Citrus unshiu* with an inline squeezer and filtering through a finisher (0.5 mm screen) was subjected to a light centrifugation (2,500 x g · minute) using a centrifuge (BRPX 417, manufactured by Alfa Laval). Supernatant of the light centrifugation was subjected to a heavy centrifugation (4,000 x g · minute) using the same centrifuge and the precipitates were collected. 1.2 kg of a powder prepared by freeze-drying 8 kg of this squeezing residue (orange juice minute pulp, 85% in moisture percentage) and pulverizing using a grinding machine, was stirred for 2 hours by adding 4.5 liters of ethanol thereto, thereby carrying out the extraction. After filtration, the ethanol fraction was recovered and ethanol was evaporated using an evaporator, 300 ml of water was added and stirred and then the water-insoluble components were recovered by filtration, and the same operation was repeated twice. As a result, 10 g of *Citrus unshiu* extract was obtained. In this extract were contained 4% by weight of cryptoxanthin and 20% by weight of sphingolipid.

### Test Example 1-1

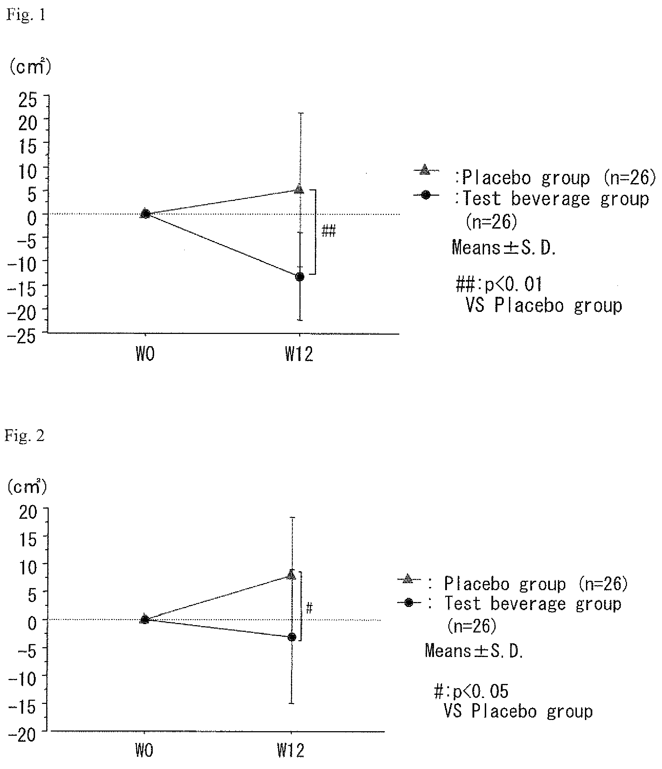

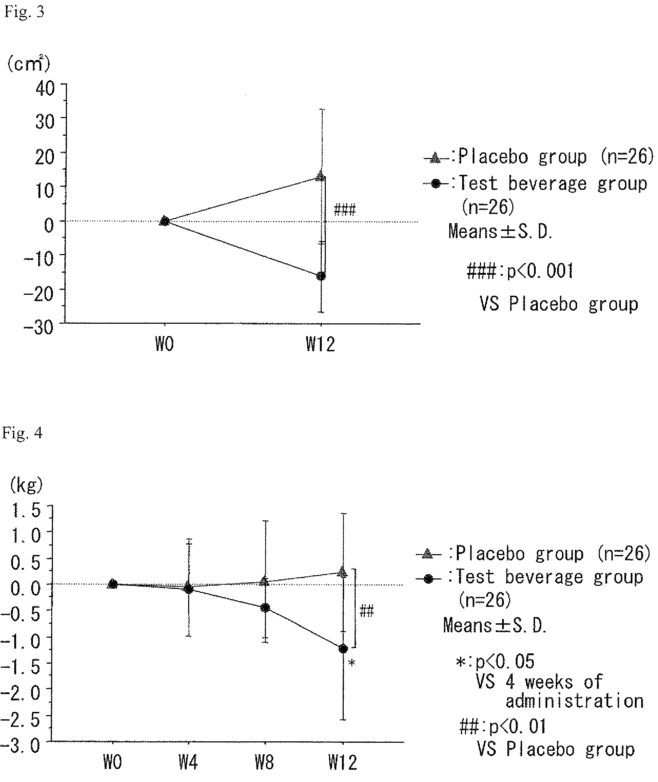

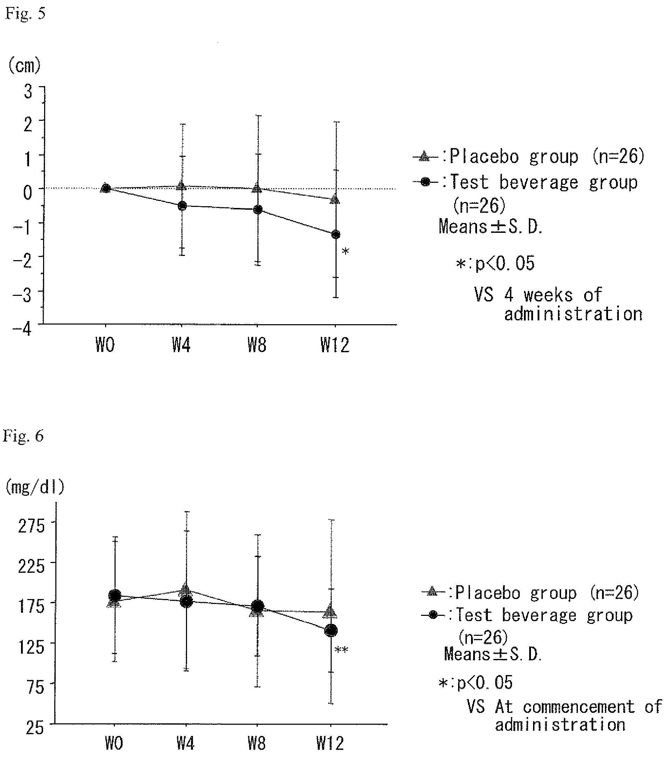

For 26 moderately obese males having a BMI value of from 25 to 30 (40.7 years old in average), an emulsion preparation was produced by mixing the extract produced in Example 1, containing cryptoxanthin and sphingolipid, together with an emulsifier. This emulsion preparation was processed into juice, and two bottles per day of the juice (12.5 mg/day as the extract (β-cryptoxanthin 0.5 mg/day, a sphingolipid 2.5 mg/day)) were ingested for 12 weeks. The test was carried out by double blind method using 13 people for each group and an extract-free juice as a comparison (Comparative Example 1-1). CT-scan photographing was carried out mainly on the L4/L5 inter-vertebral disk crossing region, and abdominal fat area, subcutaneous fat area and total fat area were measured using a visceral fat measuring software Fat Scan Ver 3.0 (manufactured by N 2 System). In addition, body weight, waist size and neutral fat were also measured. As a result, significant difference was found in the abdominal fat area, subcutaneous fat area, total fat area, body weight, waist size and neutral fat, for Comparative Example 1-1 or before the commencement of ingestion. Respective results are shown in Fig. 1, Fig. 2, Fig. 3, Fig. 4, Fig. 5 and Fig. 6. Evidently, reducing action was found on the body fat such as visceral fat, subcutaneous fat and the like and neutral fat. In addition, as a further result of the body fat reduction, reduction of body weight and waist size was also found.

### Test Example 1-2

Female mice of KK-Ay/TacJc1 line (6 weeks of age, manufactured by CLEA Japan Inc.), established as a diabetes mellitus type II model animal, were divided into groups of 6 animals per group, and β-cryptoxanthin (95% in purity, manufactured by SHIKOKU YASHIMA PURE CHEMICALS CO., LTD.) and glucosylceramide (3% in purity, konnyaku ceramide manufactured by UNITIKA, LTD.) were mixed with a high fat feed, at the ratios of 0.2% and 4% respectively, and subjected to free ingestion together with water. As the control group, tests were carried out by providing the mice with a high fat feed (CLEA Rodent Diet Quick Fat, manufactured by CLEA Japan Inc) to which nothing was added (Comparative Example 1-2), the high fat feed to which 0.2% of β-cryptoxanthin alone was added (Comparative Example 1-3) and the high fat feed to which 4% of glucosylceramide alone was added (Comparative Example 1-4).

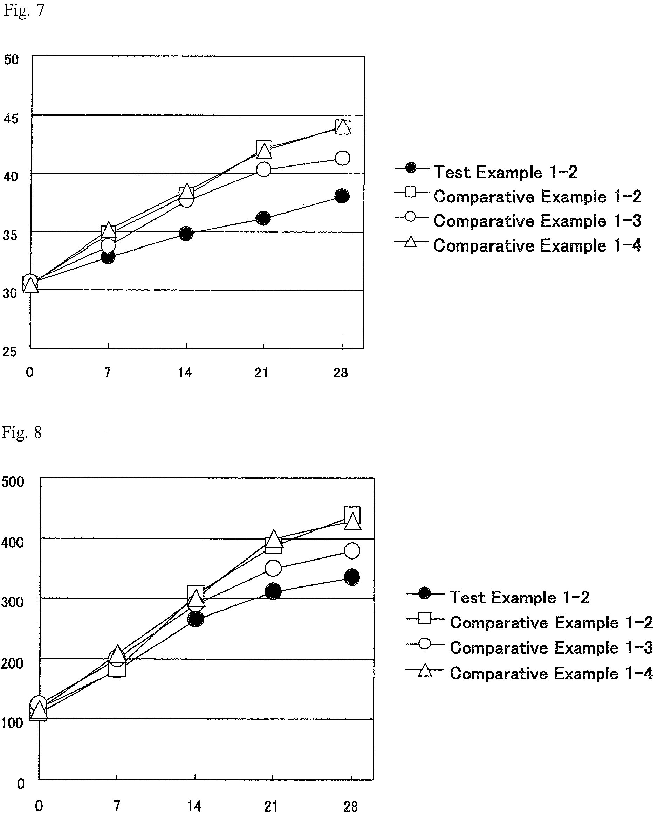

The administration was carried out for 4 weeks, and body weight and triglyceride content were measured before the administration and on the 7th, 14th, 21 st and 28th days after the administration. In addition, the mice were sacrificed on the 29th day after the commencement of administration, and the visceral fats, namely abdominal white fat tissues, were collected and their weights were compared. The results are shown in Fig. 7, Fig. 8 and Fig. 9, respectively.

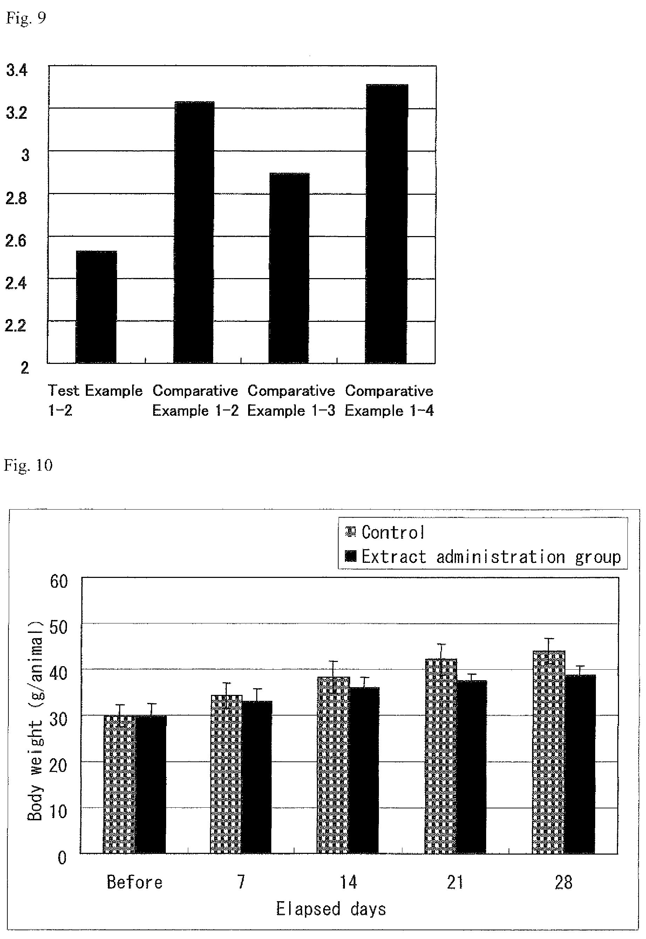

Regarding the body weight, there was a tendency of reducing body weight in the group of Test Example 1-2, from the 7th day, in comparison with Comparative Example 1-2 and Comparative Example 1-4, and significantly reduced on the 14th, 21st and 28th days (Fig. 7). It was confirmed that the triglyceride content was significantly reduced on and after the 21st day in the Test Example 1-2 (Fig. 8). Also, the abdominal fat tissue weight was significantly reduced in Test Example 1-2 and Comparative Example 1-3, in comparison with Comparative Example 1-2 and Comparative Example 1-4. In addition, it was found that the fat tissue weight of Test Example 1-2 became significantly small in comparison with Comparative Example 1-3 (Fig. 9).

From the above, it was confirmed that the composition of the present invention shows the action to reduce body fat and neutral fat by the simultaneous blending of a carotenoid and a sphingolipid, which is fairly stronger than the case of their individual use, and becomes an oral administration composition having high safety with smaller dose by the blending of both components.

### Reference Example 2

1 g of a pectinase enzyme preparation for food processing Sumizyme PX (manufactured by Shinnihon Chemicals Corporation, pectinase 5,000 units/g, arabanase 90 units/g), and 1 g of a cellulase/hemicellulase enzyme preparation, CellulaseY-NC (manufactured by Yakult Pharmaceutical Industry Co., Ltd.), were added to 800 g of a residue after squeezing fruit juice from *Citrus unshiu* (orange juice refuge, about 90% in moisture percentage), and thoroughly mixed to carry out static reaction at room temperature for 8 hours. After discarding the supernatant by centrifuging this reaction liquid, water was added thereto followed by stirring, and the supernatant was again discarded by centrifugation. This precipitate was dried using a freeze-dryer and crushed and powdered using a mixer type grinding machine, thereby obtaining the oral administration composition of the present invention. Contained in this powder were 0.5% by mass of β-cryptoxanthin (free form basis), 0.05% by mass of β-carotene and 15% by mass of hesperidin. By orally administering this composition to a type II spontaneously diabetic mouse, its effect on the visceral fat, white fat cells, was confirmed.

### Test Example 2-1

Female mice of KK-Ay/TacJc1 line (6 weeks of age: manufactured by CLEA Japan Inc.), established as a diabetes mellitus type II model animal, were divided into groups of 6 animals per group, and the composition obtained in Example 1 was mixed with a high fat feed at a ratio of 4% and subjected to free ingestion together with water. The high fat feed alone was allowed to be ingested by the control group.

The administration was carried out for 4 weeks, and the body weight was measured before the administration and on the 7th, 14th, 21st and 28th days after the administration. The result is shown in Fig. 10. From the 14th day, the body weight showed a reducing tendency and significantly reduced on the 21 st and 28th days in the administration group in comparison with the control group.

### Test Example 2-2

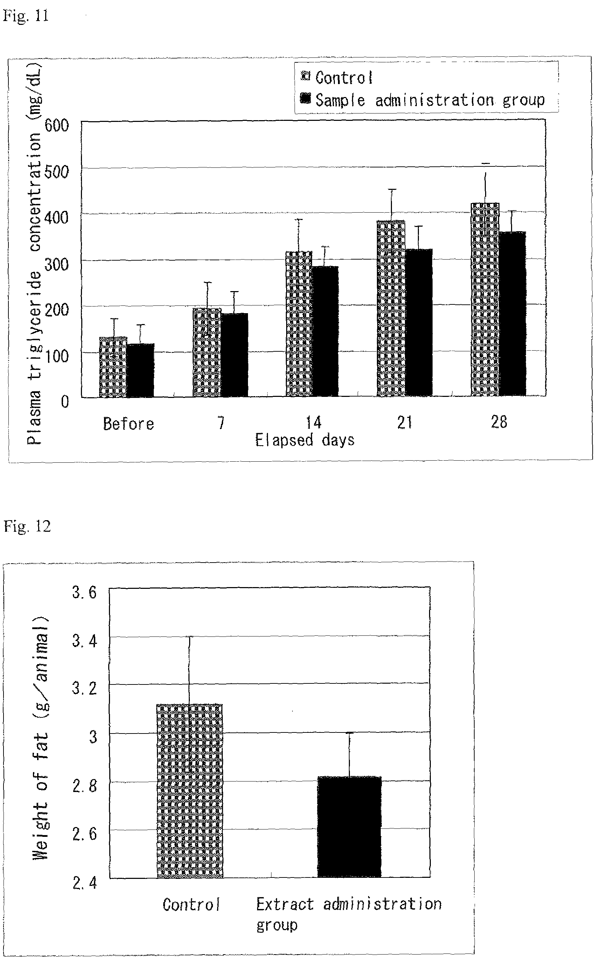

Simultaneously with the measurement of body weights of the mice used in Test Example 1-1, blood collection was carried out and the triglyceride content in blood was measured using Triglyceride E-test Wako (manufactured by Wako Pure Chemical Industries, Ltd.) The result is shown in Fig. 11. The triglyceride value was significantly reduced on the 21st and 28th days in the administration group in comparison with the control group.

### Test Example 2-3

The mice used in Test Example 2-1 were sacrificed on the 29th day after commencement of the administration, and collection of the visceral fat, namely white fat tissue, was carried out to compare their mass. The result is shown in Fig. 12. In the administration group, mass of the fat tissue was significantly reduced in comparison with the control group.

### Reference Example 3

The oral administration composition of the present invention was obtained by adding 0.02% by mass of β-cryptoxanthin (manufactured by Extra Synthase) and 0.6% by mass of α-glycosyl-hesperidin (manufactured by Toyo Sugar Refining Co., Ltd.) to the high fat feed shown in Test Example 2-1.

### Test Example 3-1

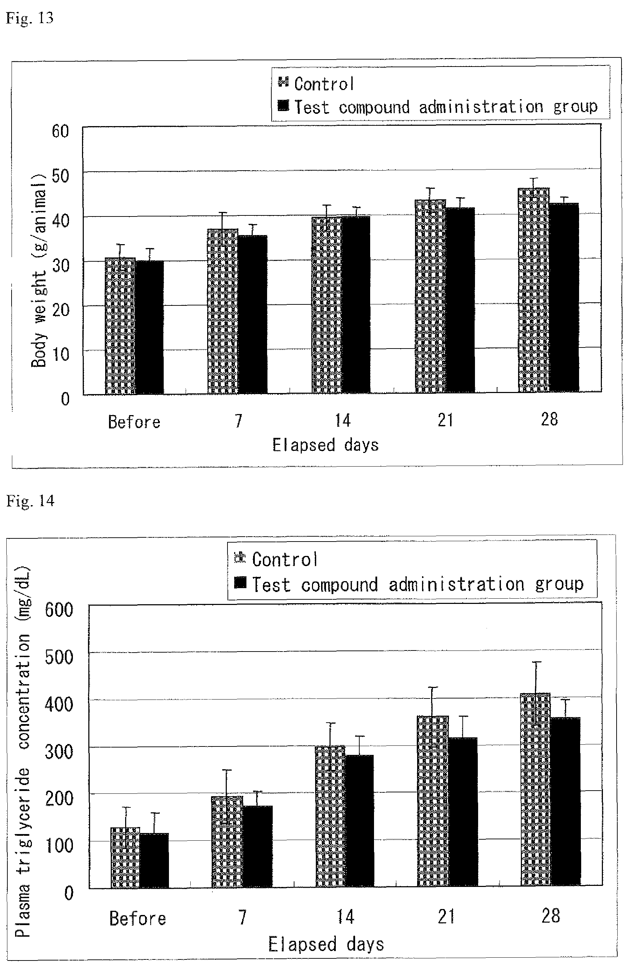

The same test of Test Example 2-1 was carried out using the composition obtained in Reference Example 3, and the result is shown in Fig. 13. In the administration group, the body weight showed a tendency to reduce from the 21 st day and was significantly reduced on the 28th day of administration, in comparison with the control group.

### Test Example 3-2

Simultaneously with the measurement of body weights of the mice used in Test Example 3-1, blood collection was carried out and the triglyceride content in blood was measured by the same method of Test Example 2-2. The result is shown in Fig. 14. The triglyceride value was significantly reduced on the 21st and 28th days in the administration group in comparison with the control group.

### Test Example 3-3

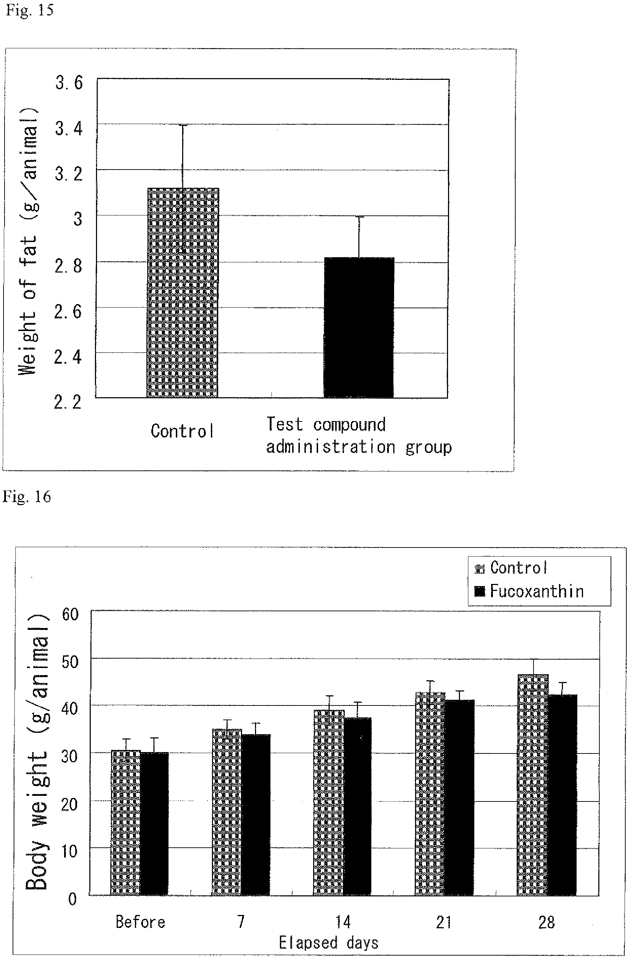

The mice used in Test Example 3-1 were sacrificed on the 29th day after commencement of the administration, and collection of the abdominal white fat tissue was carried out in the same manner as Test Example 2-3 to compare their mass. The result is shown in Fig. 15. In the administration group, the mass of the fat tissue was significantly reduced in comparison with the control group.

### Comparative Example 2

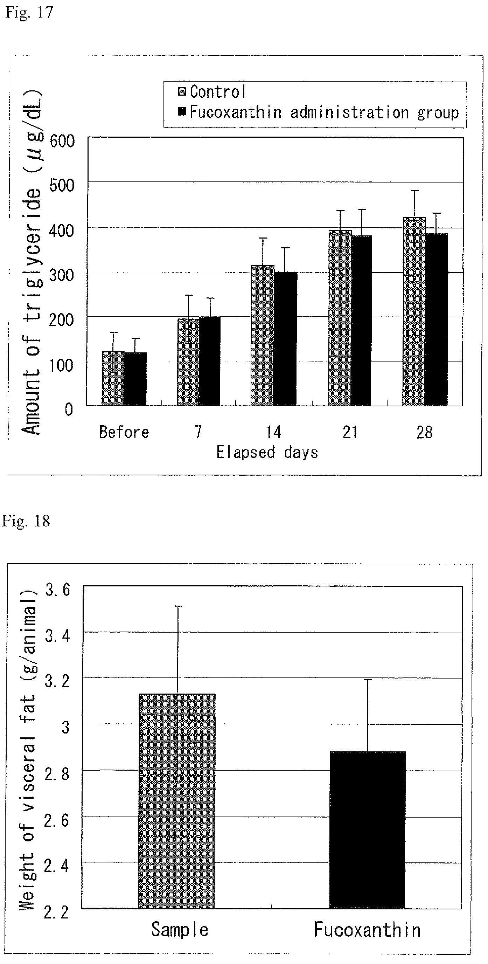

A *wakame* seaweed extract which contains fucoxanthin already known to have a visceral fat reducing action was mixed with the above-mentioned high fat feed, in such a manner that it became equivalent to the 0.02% by mass of the β-cryptoxanthin concentration contained in Test Example 2-1, and used as Comparative Example 2, and its effect was verified by carrying out the same tests as Test Examples 2-1, 2-2 and 2-3. Respective results are shown in Fig. 16, Fig. 17 and Fig. 18. Regarding the change in body weight, it showed a reducing tendency from the 21 st day and was significantly reduced on the 28th day (Fig. 16). Regarding the triglyceride content in blood, a great difference was not found in comparison with the control group (Fig. 17). Regarding the mass of abdominal white fat tissue after the 29th day, the mass of the visceral fat was significantly reduced in comparison with the control group (Fig. 18).

From the above results, it was revealed that in comparison with the *wakame* extract which contains fucoxanthin, the composition containing β-cryptoxanthin and α-glucosylhesperidin has stronger effect to reduce the body weight, the triglyceride content in blood and the mass of abdominal white fat tissue.

### Comparative Example 3

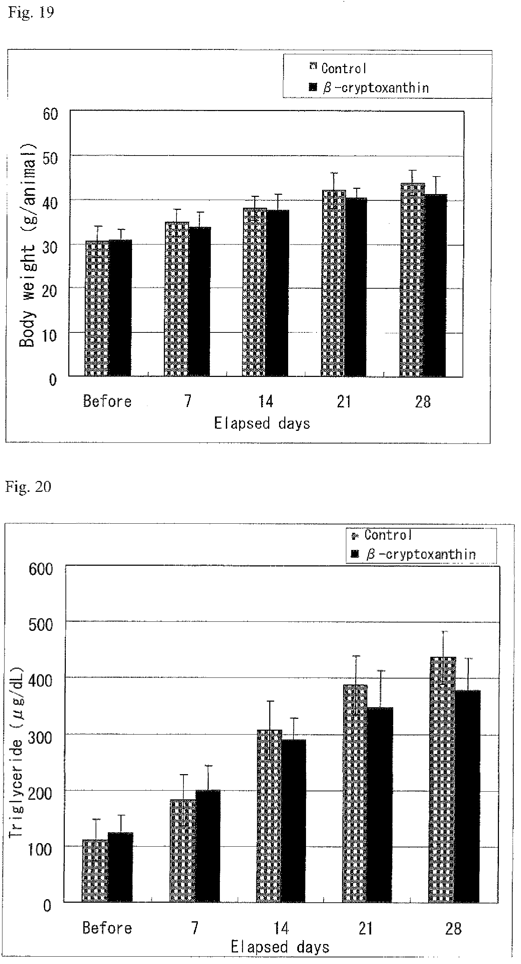

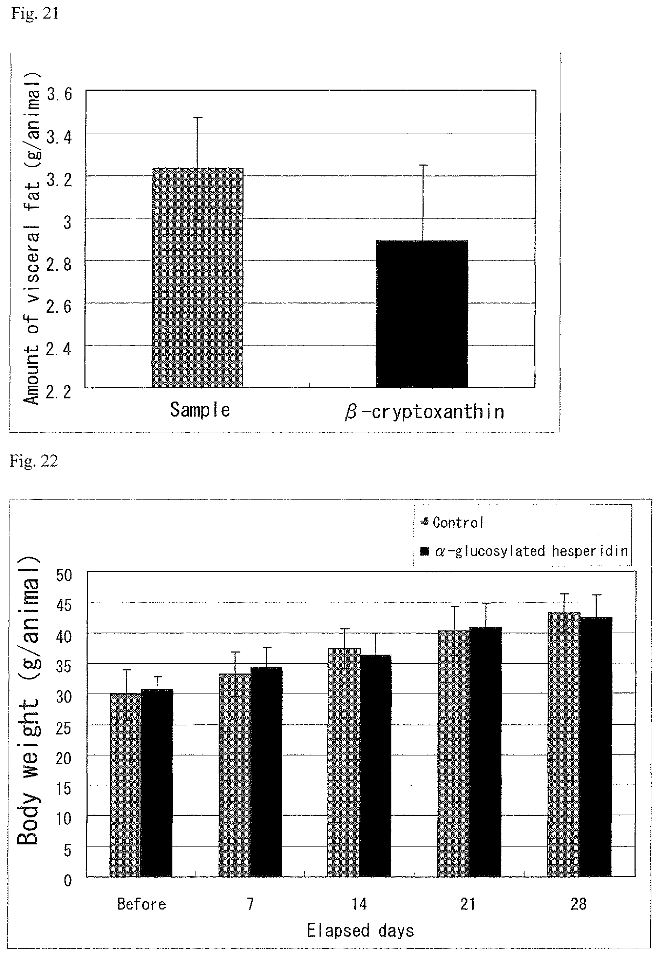

A high fat feed was prepared as Comparative Example 3 in such a manner that it contained 0.02% by mass of commercially available β-cryptoxanthin (manufactured by Extra Synthase), and its effect was verified by carrying out the same tests as Test Examples 2-1, 2-2 and 2-3. Respective results are shown in Fig. 19, Fig. 20 and Fig. 21. Regarding the change in body weight, it was significantly reduced on the 28th day in comparison with the control group (Fig. 19). Regarding the triglyceride content in blood, it was significantly reduced on the 21 st and 28th days in comparison with the control group (Fig. 20). Regarding the mass of the abdominal white fat tissue after 29 days, the mass of the fat tissue was significantly reduced in the administration group in comparison with the control group (Fig. 21).

From the above results, it was revealed that in comparison with the action of β-cryptoxanthin alone, the action of the combination use of β-cryptoxanthin and α-glucosylhesperidin has stronger effect to reduce the body weight, the triglyceride content in blood and the mass of abdominal white fat tissue.

### Comparative Example 4

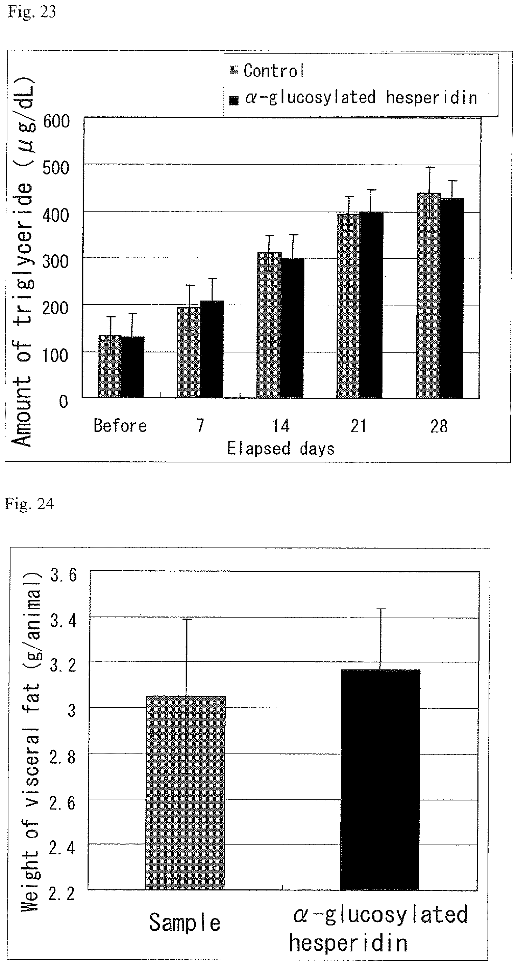

A high fat feed was prepared as Comparative Example 4 in such a manner that it contained 0.6% by mass of commercially available α-glycosylhesperidin (manufactured by Toyo Sugar Refining Co., Ltd.), and its effect was verified by carrying out the same tests as Test Examples 2-1, 2-2 and 2-3. Respective results are shown in Fig. 22, Fig. 23 and Fig. 24. Regarding the change in body weight, significant difference was not found between the administration group and control group (Fig. 22). Regarding the triglyceride content in blood, significant difference was not found between the administration group and control group (Fig. 23). Regarding the mass of abdominal white fat tissue after 29 days, reduction of the visceral fat was not found between the administration group and test group (Fig. 24).

From the above results, it was revealed that in comparison with the action of α-glucosylhesperidin alone, the action of combination use of β-cryptoxanthin and α-glucosylhesperidin has stronger effect to reduce the body weight, the triglyceride content in blood and the mass of abdominal white fat tissue.

### Reference Example 4

The powder composition produced in Reference Example 2 was thoroughly mixed with the following materials at the respective blending ratios, and then formed and baked in an oven to produce a cracker having orange flavor.

| | | |
|---|---|---|
| Materials | Powder composition | 5% |
| | Wheat flour | 50% |
| | Sugar | 20% |
| | Liquid egg | 5% |
| | Butter | 18.5% |
| | Calcium carbonate | 1% |
| | Table salt | 0.5% |

### Reference Example 5

The powder composition produced in Reference Example 2 was thoroughly mixed with the following materials at the respective blending ratios and then subjected to tablet making to produce tablets.

| | | |
|---|---|---|
| Materials | Powder composition | 40% |
| | Egg shell calcium | 5% |
| | Crystalline cellulose | 10% |
| | Reducing maltose | 43% |
| | Sucrose fatty acid ester | 2% |

### Reference Example 6

The powder composition produced in Reference Example 2 was thoroughly mixed with the following materials at the respective blending ratios to produce a feed for hog raising. In this connection, 1 kg of the vitamin-mineral mixture contains 1.5 mg of thiamine nitrate, 10.5 mg of riboflavin, 0.75 mg of pyridoxine chloride, 9 mg of nicotinamide, 16.4 mg of calcium D-pantothenate, 86.4 mg of choline chloride, 10,000 IU of vitamin A, 32,000 IU of vitamin D, 10 mg of tocopherol acetate, 50 mg of Mn, 50 mg of Fe, 10 mg of Cu, 50 mg of Zn and 1 mg of I.

| | | |
|---|---|---|
| Materials | Powder composition | 2% |
| | Corn | 65% |
| | Soybean meal | 16.5% |
| | Wheat bran | 15.2% |
| | Calcium carbonate | 0.6% |
| | Dicalcium phosphate | 0.05% |
| | Sodium chloride | 0.3% |
| | Vitamin-mineral mixture | 0.35% |

### Reference Example 7

1 L of ethanol was added to 100 g of the powder composition produced in Reference Example 2 and stirred for 1 hour, and then the ethanol-soluble components β-cryptoxanthin and the like) were extracted and isolated by filtration. By concentrating this fraction through the evaporation of ethanol using an evaporator, a *Citrus unshiu* extract was obtained. This extract contained 5% of β-cryptoxanthin (as free form) and 0.5% of β-carotene.

By thoroughly mixing this extract with the following materials so as to be 100 ml, a drink preparation was produced.

| | | |
|---|---|---|
| Materials | *Citrus unshiu* extract | 10 mg |
| | Glucose fructose syrup | 500 mg |
| | Nicotinamide | 20 mg |
| | Vitamin B1 nitrate | 5 mg |
| | Vitamin B2 phosphate | 5 mg |
| | Vitamin B6 | 5 mg |
| | Anhydrous caffeine | 50 mg |
| | α-Glucosylhesperidin | 5 mg |

### Reference Example 8

The *Citrus unshiu* extract produced in Reference Example 7 and the following the materials were thoroughly mixed so as to be 100 ml to produce jelly having orange flavor.

| | | |
|---|---|---|
| Materials | *Citrus unshiu* extract | 20 mg |
| | Granulated sugar | 20 g |
| | Gelatin | 4 g |
| | α-Glucosylhesperidin | 10 mg |

### Reference Example 9

The *Citrus unshiu* extract produced in Reference Example 7 and the following the materials were thoroughly mixed to produce a low calorie ice cream enriched with β-cryptoxanthin.

| | | |
|---|---|---|
| Materials | Citrus unshiu extract | 20 mg |
| | Tofu | 80 g |
| | Milk | 30 ml |
| | Egg yolk | 25 g |
| | Corn starch | 20 g |
| | Reduced starch syrup | 10 g |
| | Suclarose | 40 mg |
| | α-Glucosylhesperidin | 20 mg |
| | β-Cryptoxanthin | 2 mg |
| | Vanilla essence | 0.01 mg |

### Industrial Applicability

The present invention relates to a composition having high safety, which comprises a composition containing a carotenoid and a sphingolipid, that shows the effect as a body fat reducing action, a neutral fat reducing action and the like, and can be ingested as a food, a pharmaceutical or a feed.

## Claims

1. An oral administration composition comprising a carotenoid and a sphingolipid for use in a method of reducing body fat, wherein:
the carotenoid is cryptoxanthin and/or a fatty acid ester thereof; and
the carotenoid and/or sphingolipid is derived from a citrus fruit selected from iyokan, (Citrus iyo), Citrus unshiu, kabosu (Citrus sphaerocarpa), Kawabata, kishu mikan (Citrus kinokuni), kiyomi, kinkan (Citrus fortunella), grape fruit, gekkitsu (Murraya paniculata), sanpokan (Citrus sulcata), shekwasha (Citrus depressa Hayata), jabara (Citrus jabara), sweety (oroblanco), sudachi (Citrus sudachi), daidai (Citrus sinensis var. daidai), tachibana (Citrus tachibana), dekopon, natsu daidai, hassaku (Citrus hassaku), Valencia orange, banpaku yuzu (Citrus grandis, Citrus maxima), hyuga natsu (Citrus tamurana hort, ex T.Tanaka), buntan (Citrus maxima), ponkan (Citrus reticulata), mandarin orange, yatsushiro, yuzu (Citrus junos), lime (Citrus aurantifolia) and karatachi (Poncirus trifoliate).

2. A composition for use according to Claim 1, wherein the citrus fruit is *Citrus unshiu.*

3. A composition for use according to Claim 1 or Claim 2, wherein the composition is part of a food.

4. A composition for use according to Claim 1 or Claim 2, wherein the composition is part of a pharmaceutical or a feed.

## Patentansprüche

1. Zusammensetzung zur oralen Verabreichung, umfassend ein Carotinoid und ein Sphingolipid, zur Verwendung in einem Verfahren zur Reduzierung von Körperfett, wobei:
das Carotinoid Cryptoxanthin und/oder ein Fettsäureester davon ist; und
das Carotinoid und/oder Sphingolipid von einer Zitrusfrucht abgeleitet ist/sind, ausgewählt aus Iyokan, (Citrus iyo), Citrus unshin kabosu (Citrus sphaerocarpa), Kawabata, Kishu mikan (Citrus kinokuni), Kiyomi, Kinkan (Citrus fortunella), Grapefruit, Gekkitsu (Murraya paniculata), Sanpokan (Citrus sulcata), Shekwasha (Citrus depressa Hayata), Jabara (Citrus jabara), Sweety (Oroblanco), Sudachi (Citrus sudachi), Daidai (Citrus sinensis var. daidai), Tachibana (Citrus tachibana), Dekopon, Natsu daidai, Hassaku (Citrus hassaku), Valencia-Orange, Banpaku yuzu (Citrus grandis, Citrus maxima), Hyuga natsu (Citrus tamurana hort, ex T. Tanaka), Buntan (Citrus maxima), Ponkan (Citrus reticulata), Mandarinorange, Yatsushiro, Yuzu (Citrus junos), Limette (Citrus aurantifolia) und Karatachi (dreiblättriger Poncirus).

2. Zusammensetzung zur Verwendung gemäss Anspruch 1, wobei die Zitrusfrucht Citrus unshiu ist.

3. Zusammensetzung zur Verwendung gemäss Anspruch 1 oder Anspruch 2, wobei die Zusammensetzung ein Teil eines Nahrungsmittels ist.

4. Zusammensetzung zur Verwendung gemäss Anspruch 1 oder Anspruch 2, wobei die Zusammensetzung ein Teil eines Pharmazeutikums oder eines Futtermittels ist.

## Revendications

1. Composition pour administration orale comprenant un caroténoïde et un sphingolipide pour utilisation dans un procédé de réduction de la graisse corporelle, dans laquelle :
le caroténoïde est la cryptoxanthine et/ou l'un de ses esters d'acides gras ; et
le caroténoïde et/ou le sphingolipide sont dérivés d'un agrume choisi parmi l'iyokan (Citrus iyo), Citrus unshiu, le kabosu (Citrus sphaerocarpa), Kawabata, le kishu mikan (Citrus kinokuni), le kiyomi, le kinkan (Citrus fortunella), le raisin, le gekkitsu (Murraya paniculata), le sanpokan (Citrus sulcata), le shekwasha (Citrus depressa Hayata), le jabara (Citrus jabara), le sweetie (oroblanco), le sudachi (Citrus sudachi), le daidai (Citrus sinensis var. daidai), le tachibana (Citrus tachibana), le dekopon, le natsu daidai, le hassaku (Citrus hassaku), l'orange Valencia, le banpaku yuzu (Citrus grandis, Citrus maxima), l'hyuga natsu (Citrus tamurana Hort. ex Tanaka), le buntan (Citrus maxima), le ponkan (Citrus reticulata), la mandarine, l'yatsushiro, le yusu (Citrus junos), le citron vert (Citrus aurantiifolia) et le karatachi (Poncirus trifoliate).

2. Composition pour utilisation selon la revendication 1, dans laquelle l'agrume est *Citrus unshiu.*

3. Composition pour utilisation selon la revendication 1 ou la revendication 2, la composition faisant partie d'un aliment.

4. Composition pour utilisation selon la revendication 1 ou la revendication 2, la composition faisant partie d'un agent pharmaceutique ou d'un aliment pour animaux.
